# NEW EUROPEAN PATENT SPECIFICATION

(11) **EP 2 933 852 B2**
(45) Date of publication and mention of the opposition decision: **06.09.2023**
(45) Mention of the grant of the patent: 17.05.2017
(21) Application number: 14165200.8
(22) Date of filing: 17.04.2014
(51) Int. Cl.: H10K 50/15, H10K 50/18, H10K 101/10, H10K 85/60

(54) **Phosphorescent OLED and hole transporting materials for phosphorescent OLEDs**
Phosphoreszente OLED und Lochtransportmaterialien für phosphoreszierende OLED
Diode électroluminescente organique phosphorescente et matières de transport de trous pour diodes électroluminescentes phosphorescentes

(43) Date of publication of application: 21.10.2015
(73) Proprietor: Novaled GmbH, 01099 Dresden (DE)
(72) Inventor: Zöllner, Mike, 01217 Dresden (DE); Köhler, Martin, 01307 Dresden (DE); Wallikewitz, Bodo, 01099 Dresden (DE)
(74) Representative: Bittner, Thomas L.

(56) References cited:
- EP-A2- 0 687 668
- WO-A1-01/93642
- WO-A1-2013/182046
- WO-A1-2016/078738
- CN-A- 101 088 992
- JP-B2- 3 824 385
- KR-A- 20110 100 877
- KR-A- 20110 111 095
- US-A1- 2008 124 573
- US-A1- 2008 124 573
- US-A1- 2012 217 492
- US-A1- 2014 361 269
- US-A1- 2015 187 850
- Yersin H.: "Highly Efficient OLEDs with Phosphorescent Materials", 2008, Wiley-VCH, Weinheim pages 1-4,
- G. P. Moss ET AL: "Glossary of class names of organic compounds and reactive intermediates based on structure", Pure & Appl. Chem., vol. 67, no. 8/9, 1 January 1995, pages1307 and 1320
- 1UPAC, "Compendium of Chemical Terminology" International Union of Pure and Applied Chemistry (24.02.2014)
- March, Jerry, "Advanced Organic Chemistry: Reactions, Mechanism, and Structure", John Wiley & Sons, 1992, Ed. 4, p. 40-48
- Streitwieser, Andrew et al., "Introduction to Organic Chemistry", Macmillian Publishing Company, 1992, Ed. 4, p. 604-610
- Tokito, Shizuo et al., "Phosphorescent Organic Light-emitting Devices: Triplet Energy Management", Review - Electrochemistry, Vol. 1, No. 76, p. 24-31, 2008
- Rompp Chcmic Lcxikon (1995), definition of "Aryl"
- machine translation of D4

## Description

The present invention relates to phosphorescent organic light-emitting devices, and to compounds which may be used in such devices, especially in hole transporting and/or electron blocking layers thereof.

In OLEDs, the electroluminescence (EL) property of certain organic materials is used. In EL devices, suitable charge carriers are formed under application of a voltage across the device. Recombination of these charge carriers results in an excited state, which relaxes to the ground state under light emission. To increase the efficiency, the organic light-emitting diodes very often have, besides the emission layer, also charge transporting layers which are responsible for transport of negative and positive charge carriers into the emission layer. These charge transporting layers are grouped, depending on the charge carrier transported, into hole conductors and electron conductors. A quite similar set of layers is known for photovoltaic devices, such as organic solar cells. Organic semiconducting devices having several layers are produced by known methods, for example evaporation under vacuum or deposition from solution.

In other words, in case of organic light-emitting diodes, light is produced and emitted by the injection of charge carriers, electrons from one side, holes from the other, from the contacts into adjacent organic layers as a result of an externally applied voltage, subsequent formation of excitons (electron-hole pairs) and their radiative recombinationin a recombination zone.

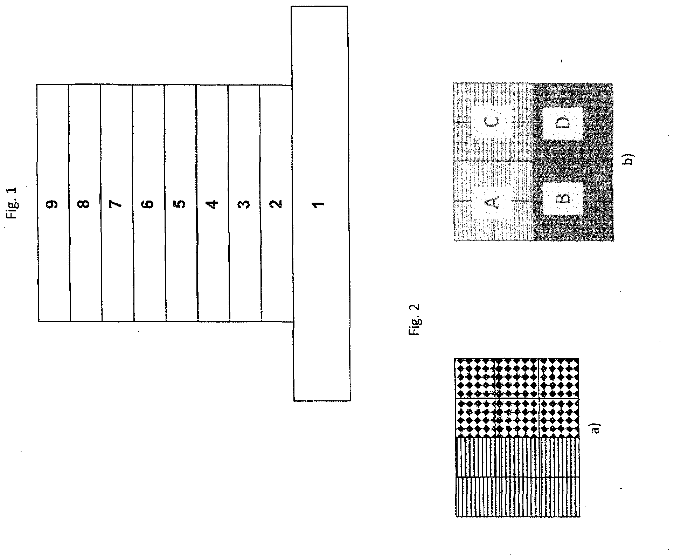

The state-of-the-art OLED structure with the positive electrode (anode) adjacent to the substrate is schematically shown in Fig. 1, wherein the numbers 1-9 denominate the following layers:
1. Transparent substrate
2. Transparent anode as bottom, hole injecting electrode
3. Hole-injecting layer
4. Hole-transporting layer (HTL)
5. Light-emitting layer (EML)
6. Electron-transporting layer (ETL)
7. Electron-injecting layer (EIL)
8. Cathode as top electrode (usually a metal with low work function, electron-injecting)
9. Encapsulation to protect from moisture, oxygen and other outside factors which may affect stability or efficiency.

While this description represents the most common case, often several layers may be omitted, or else one layer may fulfill several functions.

An important property of organic semiconducting materials is their conductivity. The conductivity of a thin layer sample can be measured by, for example, by the two-point method. A voltage is applied to the thin layer and the current flowing through the layer is measured. The measured resistance or conductivity, respectively, can be calculated from the geometry of the contacts and the thickness of the layer of the sample.

In an OLED, the operational voltage (or, more exactly, the overall electrical resistance) is determined not only by resistances and thicknesses of particular layers, but also by energetic barriers for charge carrier injection from a particular layer to the adjacent one. The power efficiency of the device (conversion of the electrical power in the light flux at the given wavelength or in the given colour range) depends on (a) Joule losses caused by the overall resistance and on (b) the efficiency of conversion of charge carriers into photons. The latter depends predominantly on the charge carrier (electron-hole) balance and on the quantum efficiency of radiative recombination of the electron-hole pairs (excitons) in the device.

There has been steady effort to develop materials and OLED designs which minimize Joule losses, improve charge carrier balance and maximize the quantum efficiency. Joule losses have been reduced significantly through improved charge injecting layers and the introduction of electrically doped charge transporting layers (see e.g. WO2003/070822 or WO2005/086251 and references cited therein). Specific charge injecting and blocking layers can also improve the charge carrier balance. A further improvement in quantum efficiency has been obtained through phosphorescent emitters, which allow in theory 100 % efficiency as all excitons formed can decay radiatively. By comparison, in fluorescent emitters triplet and singled excitons are formed, but only singlet excitons can decay radiatively. Therefore, the quantum efficiency in fluorescent emitters is less than 100 %.

A number of materials used for preparing hole transport layers and/or electron/exciton blocking layers are known.

However, the OLED efficiency is still significantly below its theoretical limits and many other OLED-performance parameters like luminosity and lifetime can be also further improved.

From a practical point of view, it is advantageous that one matrix compound is suitable for various OLED designs, e.g. in phosphorescent as well as fluorescent OLEDs, and in both electrically doped hole injecting and/or hole transporting layers as well as in electrically undoped electron blocking layers.

It is therefore an object of the present invention to provide improved phosphorescent OLEDs having lower operating voltage and/or higher efficiency than devices using hole transporting and electron blocking matrices according to the state of the art. Another object of the inventtion is providing new compounds which can be used as matrix materials for hole-transporting layers and/or electron/exciton blocking layers which overcome the drawbacks of the prior art and can especially be used in phosphorescent OLEDs.

This object is achieved by an OLED comprising between anode and cathode at least one emitting layer comprising a phosphorescent emitter and at least one hole transporting layer comprising a compound represented by general formula (I) 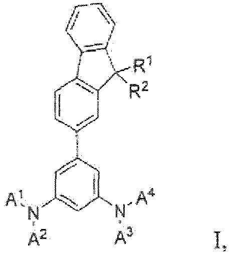
wherein R¹ and R² can be independently selected from hydrogen, C₁-C₂₀ alkyl or C₃-C₂₀ cycloalkyl, C₇-C₂₀ arylalkyl, C₆-C₁₂ aryl,
A¹, A², A³ and A⁴ are independently selected from C₆-C₂₀ aryl, and the substituents R¹ and R² may be linked so that they form a ring.

The alkyl substituents can be saturated or unsaturated, straight or branched. The cycloalkyl or cycloalkoxy substituent may be saturated or unsaturated, monocyclic or polycyclic. The overall C atom count in a substituent includes possible alkyl substitution, branching and/or occurrence of cyclic structures within the substituent. Preferably, the overall C atom count in the compound (I) does not exceed 150. More preferably, the overall C atom count in any substituent selected from R¹, R², A¹, A², A³ and A⁴ does not exceed 20. Most preferably, the overall C atom count in any substituents selected from R¹, R², A¹, A², A³ and A⁴ does not exceed 12.

In a preferred embodiment, in each pair of aryl substituents attached to the same nitrogen, at least one aryl selected from A¹ and A² and at least one aryl selected from A³ and A⁴ are C₆-C₁₅ aryls. In a more preferred embodiment, substituents A¹, A², A³ and A⁴ are C₆-C₁₅ aryls. In another preferred embodiment, aryl substituents A¹, A², A³ and A⁴ are selected from phenyl, tolyl, xylyl, trimethylphenyl, tert-butylphenyl, 1,1'-biphenyl-yl, naphtyl and 9H-fluorenyl. In more preferred embodiment, aryl substituents A¹, A², A³ and A⁴ are selected from 1,1'-biphenyl-yl and 9H-fluorenyl. In an even more preferred embodiment, 1,1'-biphenyl-yl is 1,1'-biphenyl-4-yl and fluorenyl is 9,9'-dimethyl-9H-fluoren-2-yl. In another preferred embodiment, R¹ and R² are independently selected from C₁-C₁₂ alkyl. Preferred are also all possible combinations of preferred embodiments mentioned above.

It is preferred that the layer comprising compound (I) is located between the emitting layer and the anode. It is further preferred that at least one layer containing the compound of formula (I) is electrically doped.

More preferably, the electrically doped layer containing compound of formula (I) is adjacent to another, electrically undoped layer comprising compound of formula (I). Even more preferably, the undoped layer comprising compound (I) serves as electron blocking layer.

The term "electrical doping" means generally an improvement of electrical properties, especially the electrical conductivity, in the electrically doped semiconducting material if compared with an undoped matrix material. More detailed explanation of current theory and various examples of electrical doping are available in many published patent documents, e.g. WO2014/037512.

In one yet preferred embodiment, the undoped layer serves as both electron-blocking and triplet exciton blocking layer.

A further object is achieved by compound represented by general formula (I) 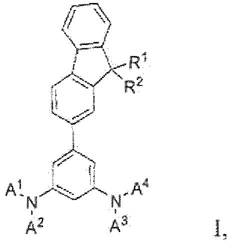
wherein R¹ and R² can be independently selected from hydrogen, C₁-C₂₀ alkyl or C₃-C₂₀ cycloalkyl, C₇-C₂₀ arylalkyl, C₆-C₁₂ aryl,
A¹, A², A³ and A⁴ are independently selected from C₆-C₂₀ aryl, and the substituents R¹ and R² may be linked so that they form a ring.

The alkyl substituents can be saturated or unsaturated, straight or branched. The cycloalkyl or cycloalkoxy substituent may be saturated or unsaturated, monocyclic or polycyclic. The overall C atom count in a substituent includes possible alkyl substitution, branching and/or occurrence of cyclic structures within the substituent. Preferably, the overall C atom count in the compound (I) does not exceed 150. More preferably, the overall C atom count in any substituent selected from R¹, R², A¹, A², A³ and A⁴ does not exceed 20. Most preferably, the overall C atom count in any substituents selected from R¹, R², A¹, A², A³ and A⁴ does not exceed 12.

In a preferred embodiment, in each pair of aryl substituents attached to the same nitrogen, at least one aryl selected from A¹ and A² and at least one aryl selected from A³ and A⁴ are C₆-C₁₅ aryls. In a more preferred embodiment, substituents A¹, A², A³ and A⁴ are C₆-C₁₅ aryls. In another preferred embodiment, aryl substituents A¹, A², A³ and A⁴ are selected from phenyl, tolyl, xylyl, trimethylphenyl, tert-butylphenyl, 1,1'-biphenyl-yl, naphtyl and 9H-fluorenyl. In more preferred embodiment, aryl substituents A¹, A², A³ and A⁴ are selected from 1,1'-biphenyl-yl and 9H-fluorenyl. In an even more preferred embodiment, 1,1'-biphenyl-yl is 1,1'-biphenyl-4-yl and fluorenyl is 9,9'-dimethyl-9H-fluoren-2-yl. In another preferred embodiment, R¹ and R² are independently selected from C₁-C₁₂ alkyl. Preferred are also all possible combinations of preferred embodiments mentioned above.

### Detailed description of the invention

Among well-known hole-transporting materials with triarylamine and benzidine structures, some alkylaryl derivatives, for example 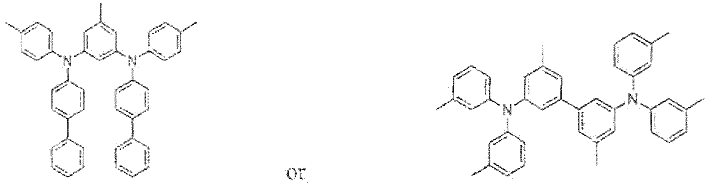 were described (see e.g. EP 687 668 or JP H03-094260). Other examples comprising a fluorenyl core can be found in US 2008/0124573. While performing detailed investigations into performance-limiting factors, it was surprisingly found by the inventors that some derivatives with similar core structures perform unexpectedly well when used in OLEDs containing a phosphorescent emitter, see PCT/EP2013/071742.

Further research helped clarifying relationships with the structure of the compounds tested and revealed that the presence of a fluorenyl substituent directly attached to the meta-diaminophenyl core is particularly advantageous and allows good performance of such compounds as HTL and/or EBL matrices in phosphorescent OLEDs.

Emitting layer, electron transporting layer, hole blocking layer, electrodes

Other parts of the inventive phosphorescent light emitting device than the inventive hole transporting and/or electron blocking layer can be prepared in various designs and from various materials described in the scientific and patent literature.

In the examples, following supporting materials were used: 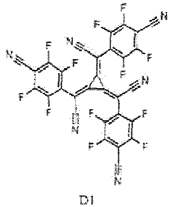 as a p-dopant (US 2010/102709), 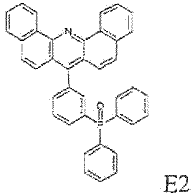 as electron-transporting matrix (WO 2013/079217), 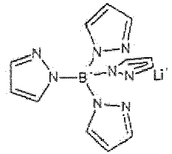 D3 as n-dopant (WO 2013/079676), 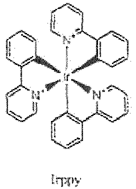 as a well-known triplet green emitter, 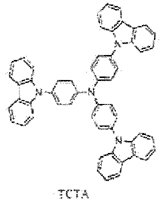 as a well-known electron blocking matrix, 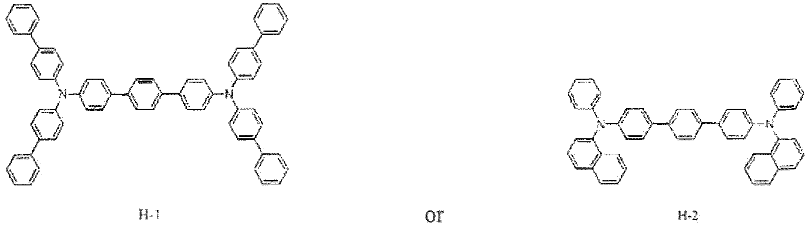 known e.g. from WO2011/134458 and US2012/223296 as comparative hole transporting matrices.

### Description of drawings

Figure 1: Schematic drawing of experimental bottom emitting phosphorescent OLED
Figure 2: a) Top view of deposition of layer 1 (p-doped inventive material (stripes), p-doped reference (dots), left; b) Top view of layer 2 after rotation of substrate by 90°, with the inventive material in the top row (fields A, C) and reference material in the bottom row (fields B, D).
Figure 3a - 3j: ¹H-NMR spectra of example compounds having formula (I) measured in CD₂Cl₂ solution, at 500.13 MHz, referenced to 5.31 ppm; 3a -FPD-1, 3b - FPD -2, 3c - FPD -3, 3d - FPD -4, 3e - FPD -5, 3f - FPD -6, 3g - FPD -7, 3h - FPD -8, 3i - FPD -9, 3j - FPD-10.

### Examples

### Synthesis of inventive compounds

### General procedure for 1-fluorenyl-3,5-dibromophenylenes (Suzuki coupling)

1,3,5-Tribromobenzene, the fluorenyboronic acid and Pd(PPh₃)₄ were dissolved in a mixture of toluene and ethanol. A degassed 2M aqueous Na₂CO₃ solution was added. The mixture was refluxed for 18-22 hours. After cooling to room temperature, the layers were separated and the organic layer was washed with water, dried and evaporated. The crude product was purified by column chromatography (SiO₂, hexane:DCM mixtures) giving the pure product. In TLC, the upper main spot was identified as the desired product and the one below as the 1,3-bis-fluorenyl bromobenzene side product.

### 2-(3,5-dibromophenyl)-9, 9-dimethyl-9H-fluorene

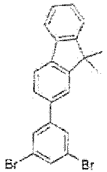

1,3,5-tribromobenzene: 126.93 g (1.2 eq, 403.2 mmol)
(9,9-dimethyl-9H-fluoren-2-yl)boronic acid: 80.00 g (1.0 eq, 336.0 mmol)
Pd(PPh₃)₄: 19.4 mg (5 mol.%, 16.8 mmol)
toluene: 960 mL
ethanol: 320 mL
2M Na₂CO₃: 640 mL
Yield: 69.4 g (48 %)
GC-MS: m/z = 426 / 428 / 430

### General procedure for secondary amines

Under an inert atmosphere, the bromoaryl component, palladium(II)acetate, cesium carbonate and 2,2'-bis(diphenylphosphino)-1,1'-binaphthyl (BINAP) were combined in a flask and dissolved in 1,4-dioxane. The primary arylamine component was added, followed by heating up the mixture to reflux and stirring for 16-48 hours until, according to TLC, the reaction was complete. The mixture was cooled to room temperature and filtered through a pad of silica gel. After washing with DCM, the organic layer was washed twice with 2M aqueous HCl, once with half-saturated Na₂CO₃ and once with water. After evaporation of the solvent, the crude product was purified by column chromatography (SiO₂, hexane:DCM mixtures). The combined fractions were evaporated to dryness. The crude product was purified by recrystallization from hexane.

### N-phenyl-[1,1'-biphenyl]-4-amine

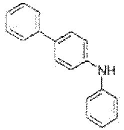

4-bromobiphenyl: 20.00 g (1.0 eq, 85.80 mmol)
aniline: 8.39 g (1.05 eq, 90.09 mmol)
palladium(II)acetate: 578 mg (3.0 mol.%, 2.57 mmol)
BINAP: 2.40 g (4.5 mol.%, 3.86 mmol)
cesium carbonate: 39.13 g (1.4 eq, 120.1 mmol)
dioxane: 200 mL
Yield: 11.05 g (52 %)
GC-MS: m/z = 245

### N-(4-(methyl)pheny)-[7,1'-biphenyl]-4-amine

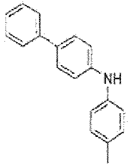

4-bromobiphenyl: 20.0 g (1.0 eq, 85.8 mmol)
4-toluidine: 9.65 g (1.05 eq, 90.1 mmol)
palladium(II)acetate: 578 mg (3.0 mol.%, 2.6 mmol)
BINAP: 2.40 g (4.5 mol.%, 3.9 mmol)
cesium carbonate: 39.14 g (1.4 eq, 120.1 mmol)
dioxane: 200 mL
Yield: 19.20 g (86 %)
GC-MS: m/z = 259

### N-(3,5-dimethylphenyl)-[1,1'-biphenyl]-4-amine

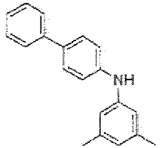

4-bromobiphenyl: 30.00 g (1.0 eq, 128.70 mmol)
3,5-dimethylaniline: 16.38 g (1.05 eq, 135.10 mmol)
palladium(II)acetate: 867 mg (3.0 mol.%, 3.86 mmol)
BINAP: 3.60 g (4.5 mod.%, 5.79 mmol)
cesium carbonate: 58.70 g (1.4 eq, 180.00 mmol)
dioxane: 300 mL
Yield: 21.34 g (60 %)
GC-MS: m/z = 273

### N-(2,4-dimethylphenyl)-[1,1'-biphenyl]-4-amine

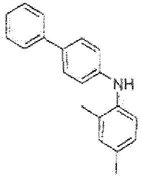

4-bromobiphenyl: 30.00 g (1.0 eq, 128.70 mmol)
3,5-dimethylaniline: 16.38 g (1.05 eq, 135.10 mmol)
palladium(II)acetate: 867 mg (3.0 mol.%, 3.86 mmol)
BINAP: 3.60 g (4.5 mol.%, 5.79 mmol)
cesium carbonate: 58.70 g (1.4 eq, 180.00 mmol)
dioxane: 300 mL
Yield: 24.40 g (69 %)
GC-MS: m/z = 273

### N-mesityl-[1,1'-biphenyl]-4-amine

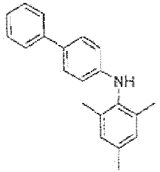

4-bromobiphenyl: 20.00 g (1.0 eq, 85.8 mmol)
mesitylamine: 12.18 g (1.05 eq, 90.1 mmol)
palladium(II)acetate: 578 mg (3.0 mol.%, 2.57 mmol)
BINAP: 2.40 g (4.5 mol.%, 3.86 mmol)
cesium carbonate: 39.13 g (1.4 eq, 120.1 mmol)
dioxane: 200 mL
Yield: 12.53 g (51 %)
GC-MS: m/z = 287

### di([1,1'-biphenyl]-4-yl)amine

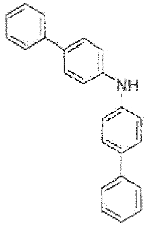

4-bromobiphenyl: 20.00 g (1.0 eq, 85.80 mmol)
biphenylamine: 15.25 g (1.05 eq, 90.09 mmol)
palladium(II)acetate: 578 mg (3.0 mol.%, 2.57 mmol)
BINAP: 2.40 g (4.5 mol.%, 3.86 mmol)
cesium carbonate: 39.13 g (1.4 eq, 120.1 mmol)
dioxane: 200 mL
Yield: 6.57 g (23 %)
GC-MS: m/z = 321

### N-(p-tolyl)naphthalen-2-amine

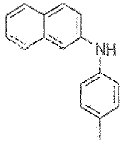

2-bromonaphthalene: 15 g (1.0 eq, 72.44 mmol)
*p*-toluidine: 11.6 g (1.5 eq, 108.6 mmol)
palladium(II)acetate: 488 mg (3.0 mol.%, 2.17 mmol)
BINAP: 2.0 g (4.5 mol.%, 3.26 mmol)
cesium carbonate: 47.20 g (2.0 eq, 144.9 mmol)
dioxane: 150 mL
Yield: 11.4 g (67 %)
GC-MS: m/z = 233

### N-(m-tolyl)naphthalen-2-amine

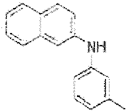

2-bromonaphthalene: 20.00 g (1.0 eq, 96.59 mmol)
m-toluidine: 15.52 g (1.5 eq, 144.89 mmol)
palladium(II)acetate: 868 mg (4.0 mol.%, 3.86 mmol)
BINAP: 3.61 g (6 mol.%, 5.79 mmol)
cesium carbonate: 62.94 g (2 eq, 193.18 mmol)
dioxane: 200 mL
Yield: 12.82 g (56 %)
GC-MS: m/z = 233

### N-(3,5-dimethylphenyl)naphthalen-2-amine

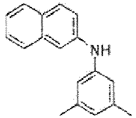

2-bromonaphthalene: 30.00 g (1.0 eq, 144.88 mmol)
3,5-dimethylaniline: 18.43 g (1.05 eq, 152.12 mmol)
palladium(II)acetate: 976 mg (3.0 mol.%, 4.35 mmol)
BINAP: 4.06 g (4.5 mol.%, 6.52 mmol)
cesium carbonate: 66.10 g (1.4 eq, 202.86 mmol)
dioxane: 300 mL
Yield: 19.90 g (55 %)
GC-MS: m/z = 247

### N-phenylnaphthalen-2-amine

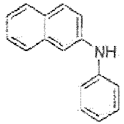

### Commercially available

### N-phenylnaphthalen-1-amine

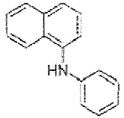

### Commercially available

### General procedure for tertiary amines

Under an inert atmosphere the dibromoaryl component, the secondary amine, bis(dibenzylidenaceton)palladium, tri-*tert*-butylphosphine and potassium-*tert*-butoxide were combined in a flask and dissolved in toluene. The mixture was stirred at 80 °C until TLC indicated complete consumption of the starting materials (usually for 90 to 210 minutes) and then cooled to room temperature. The mixture was filtered through a pad of silica gel, washed with DCM and evaporated to dryness. The crude product was stirred in boiling methanol, hexane or acetone. After cooling to room temperature, the mixture was filtered to yield the product. In case the TLC indicated still some impurities, column chromatography was used (SiO₂, hexane:DCM mixtures). Finally, all tertiary amines were purified by gradient sublimation under high vacuum (10⁻⁶ mbar) conditions.

### N1,N3-di([1,1'-biphenyl]-4-yl)-5-(9,9-dimethyl-9H-fluoren-2-yl)-N1,N3-diphenylbenzene-1,3-diamine (FPD-1)

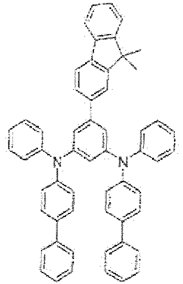

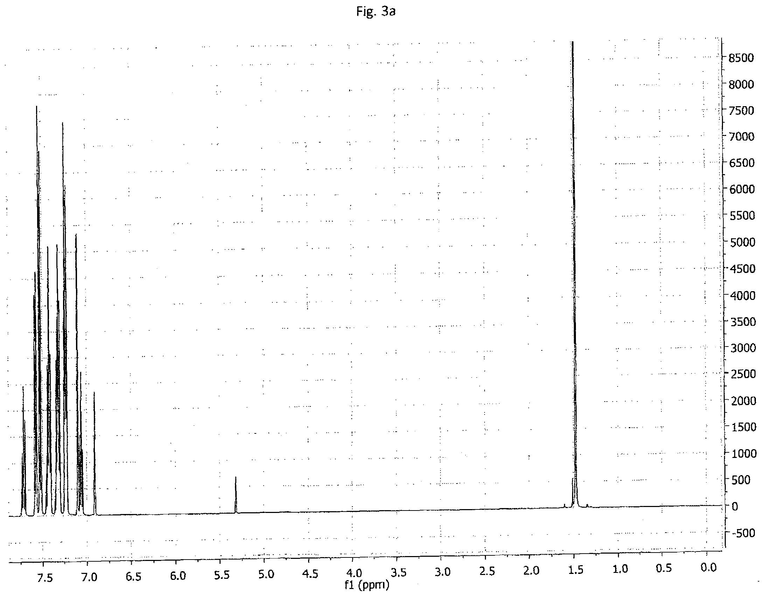

2-(3,5-dibromophenyl)-9,9-dimethyl-9H-fluorene: 3.0 g (1.0 eq, 7.01 mmol)
N-phenyl-[1,1'-biphenyl]-4-amine: 3.7 g (2.1 eq, 14.71 mmol)
bis(dibenzylidenaceton)palladium: 81 mg (2 mol.%, 0.14 mmol)
tri-*tert*-butylphosphine: 43 mg (3 mol.%, 0.21 mmol)
potassium-*tert*-butoxide: 2.36 g (3.0 eq, 21.03 mmol)
toluene: 90 mL
Yield: 4.75 g (89 %)
EI-MS: m/z = 756 [M⁺]
¹H-NMR: see Fig. 3a

### N1,N3-di([1,1'-biphenyl]-4-yl)-5-(9,9-dimethyl-9H-fluoren-2-yl)-N1,N3-di-p-tolylbenzene-1,3-diamine (FPD-2)

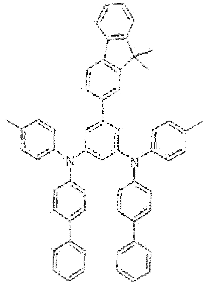

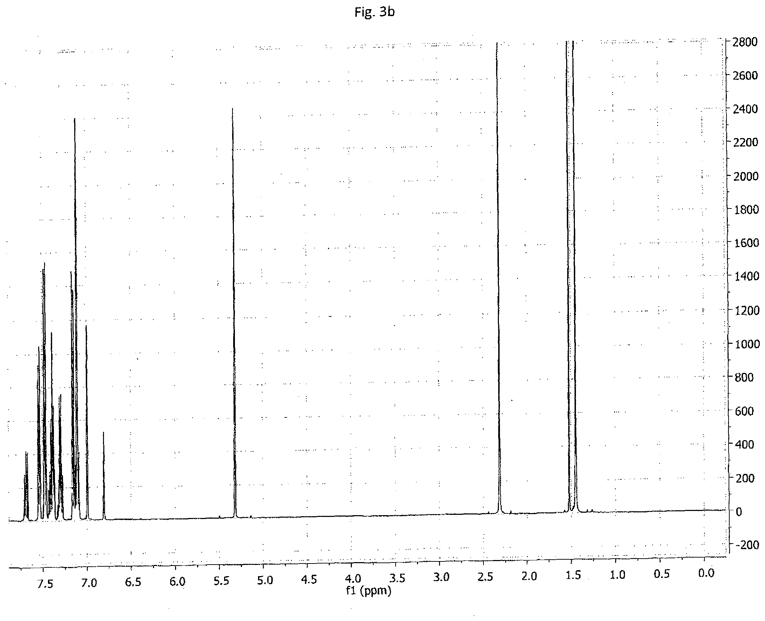

2-(3,5-dibromophenyl)-9,9-dimethyl-9H-fluorene: 3.0 g (1.0 eq, 7.01 mmol)
N-(p-tolyl)-[1,1'-bipbenyl]-4-amine: 3.82 g (2.1 eq, 14.71 mmol)
bis(dibenzylidenaceton)palladium: 81 mg (2 mol.%, 0.14 mmol)
tri-*tert*-butylphosphine: 43 mg (3 mol.%, 0.21 mmol)
potassium-*tert*-butoxide: 2.36 g (3.0 eq, 21.03 mmol)
toluene: 250 mL
Yield: 5.32 g (96 %)
HPLC-MS: m/z = 786 [M+H⁺]
¹H-NMR: see Fig. 3b

### N1,N3-di([1,1'-biphenyl]-4-yl)-5-(9,9-dimethyl-9H-fluoren-2-yl)-N1,N3-bis(3,5-dimethylphenyl)benzene-1,3-diamine (FPD-3)

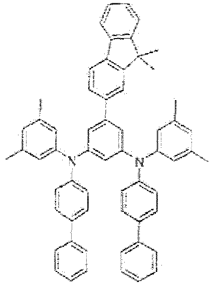

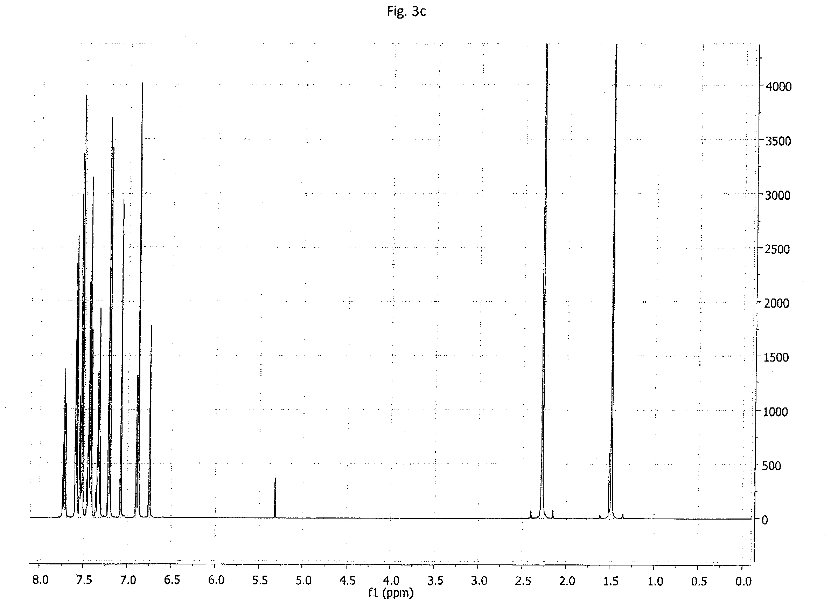

2-(3,5-dibromophenyl)-9,9-dimethy]-9H-fluorene: 11.19 g (1.0 eq, 26.13 mmol)
N N-(3,5-dimethylphenyl)-[1,1'-biphenyl]-4-amine: 15.00 g (2.1 eq, 54.87 mmol)
bis(dibenzylidenaceton)palladium: 300 mg (2 mol.%, 0.52 mmol)
tri-*tert*-butylphosphine: 158 mg (3 mol.%, 0.78 mmol)
potassium-*tert*-butoxide: 8.80 g (3.0 eq. 78.4 mmol)
toluene: 355 mL
Yield: 16.66 g (78 %)
EI-MS: m/z = 812 [M⁺]
¹H-NMR: see Fig. 3c

### N1,N3-di([1,1'-biphenyl]-4-yl)-5-(9,9-dimethyl-9H-fluoren-2-yl)-N1,N3-bis(2,4-dimethylphenyl)benzene-1,3-diamine (FPD-4)

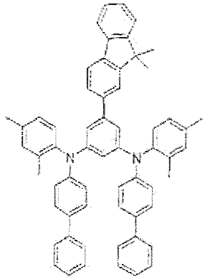

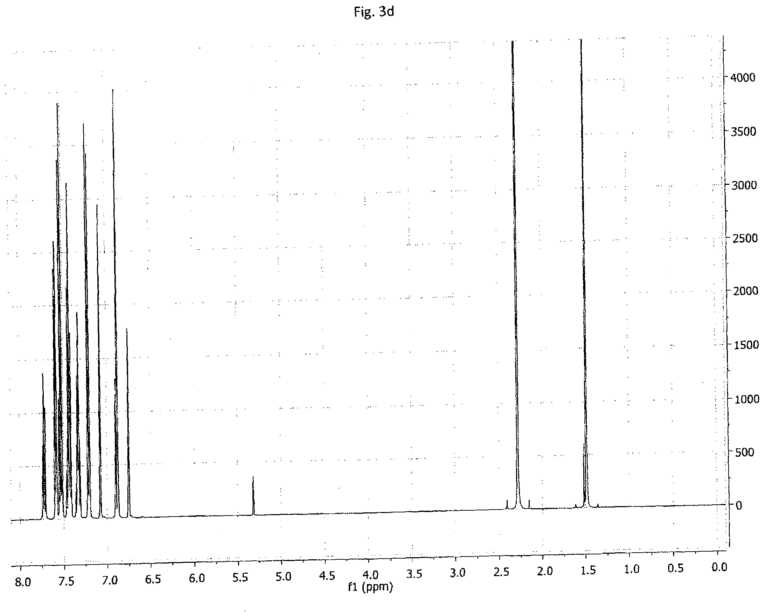

2-(3,5-dibromophenyl)-9,9-dimethyl-9H-fluorene: 3.0 g (1.0 eq, 7.01 mmol)
N-(2,4-dimethylphenyl)-[1,1'-biphenyl]-4-amine: 4.0 g (2.1 eq, 14.71 mmol)
bis(dibenzylidenaceton)palladium: 81 mg (2 mol.%, 0.14 mmol)
tri-*tert*-butylphosphine: 43 mg (3 mol.%, 0.21 mmol)
potassium-tert-butoxide: 2.36 g (3.0 eq, 21.03 mmol)
toluene: 100 mL
Yield: 4.46 g (78 %)
EI-MS: m/z = 813 [M⁺]
¹H-NMR: see Fig. 3d

### N1,N3-di([1,1'-biphenyl]-4-yl)-5-(9,9-dimethyl-9H-fluoren-2-yl)-N1,N3-dimesitylbenzene-1,3-diamine (FPD-5)

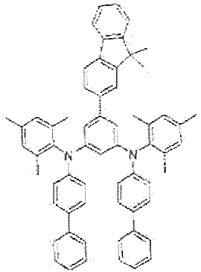

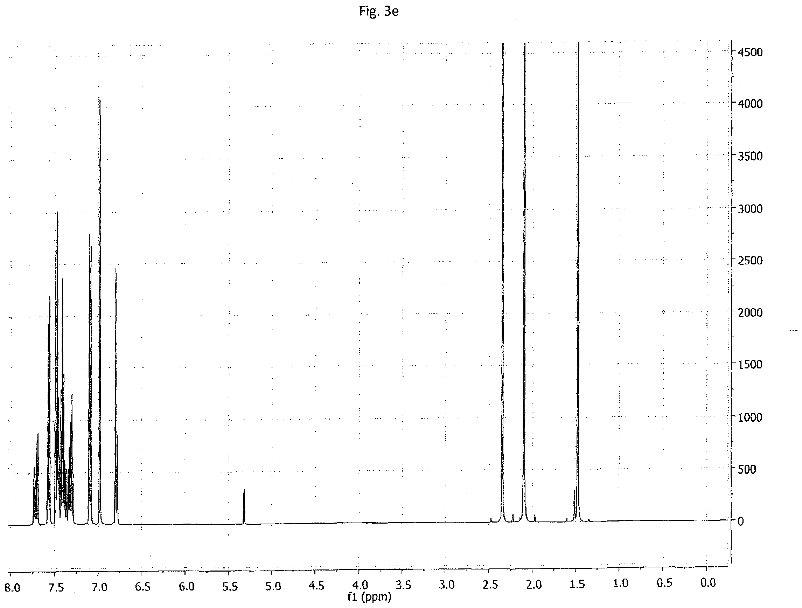

2-(3,5-dibromophenyl)-9,9-dimethyl-9H-fluorene: 10.64 g (1.0 eq, 24.85 mmol)
N-mesityl-[1,1'-biphenyl]-4-amine: 15.00 g (2.1 eq, 52.19 mmol)
bis(dibenzylidenaceton)palladium: 286 mg (2 mol.%, 0.50 mmol)
tri-*tert*-butylphosphine: 151 mg (3 mol.%, 0.75 mmol)
potassium-*tert*-butoxide: 8.36 g (3.0 eq, 74.6 mmol)
toluene: 335 mL
Yield: 12.6 g (60 %)
EI-MS: m/z = 840 [M⁺]
¹H-NMR: see Fig. 3e

### N1,N1,N3,N3-tetra([1,1'-biphenyl)-4-yl)-5-(9,9-dimethyl-9H-fluoren-2-yl)benzene-1,3-diamine (FPD-6)

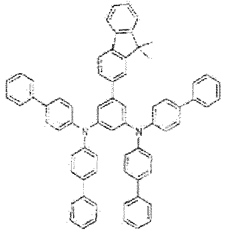

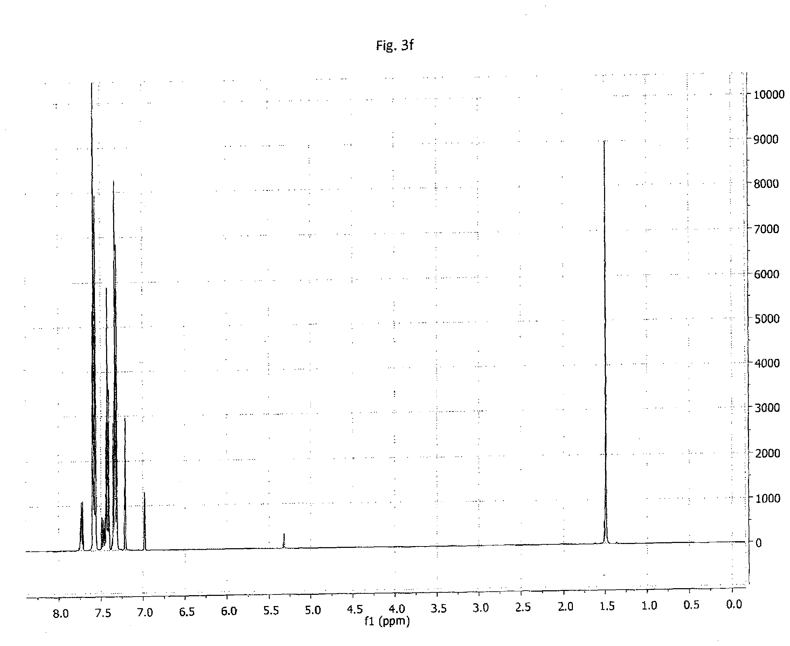

2-(3,5-dibromophenyl)-9,9-dimethyl-9H-fluorene: 3.0 g (1.0 eq, 7.01 mmol)
di([1,1'-biphenyl]-4-yl)amine: 4.7 g (2.1 eq, 14.71 mmol)
bis(dibenzylidenaceton)palladium: 81 mg (2 mol.%, 0.14 mmol)
tri-*tert*-butylphosphine: 43 mg (3 mol.%, 0.21 mmol)
potassium-*tert*-butoxide: 2.36 g (3.0 eq, 21.03 mmol)
toluene: 100 mL
Yield: 5.42 g (84 %)
EI-MS: m/z = 908.3 [M⁺]
¹H-NMR: see Fig. 3f

### 5-(9,9-dimethyl-9H-fluoren-2-yl)-N1,N3-di(naphthalen-1-yl)-N1,N3-diphenylbenzene-1,3-diamine (FPD-7)

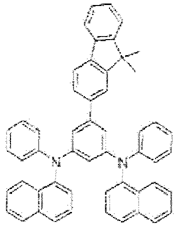

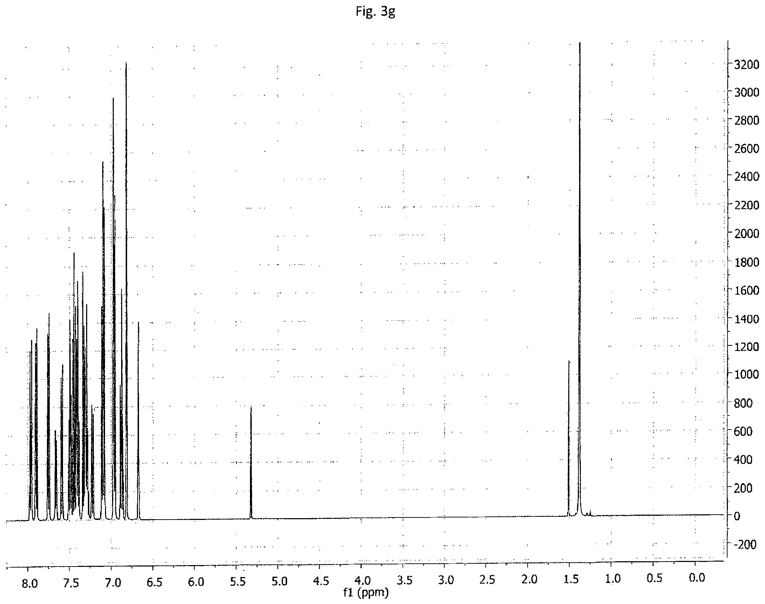

2-(3,5-dibromophenyl)-9,9-dimethyl-9H-fluorene: 3.0 g (1.0 eq, 7.01 mmol)
di([1,1'-biphenyl]-4-yl)amine: 4.7 g (2.1 eq, 14.71 mmol)
bis(dibenzylidenaceton)palladium: 81 mg (2 mol.%, 0.14 mmol)
tri-*tert*-butylphosphine: 43 mg (3 mol.%, 0.21 mmol)
potassium-*tert*-butoxide: 2.36 g (3.0 eq, 21.03 mmol)
toluene: 90 mL
Yield: 2.45 g (50 %)
EI-MS: m/z = 704 [M⁺]
¹H-NMR: see Fig. 3g

### 5-(9,9-dimethyl-9H-fluoren-2-yl)-N1,N3-di(naphthalen-2-yl)-N1,N3-diphenylbenzene-1,3-diamine (FPD-8)

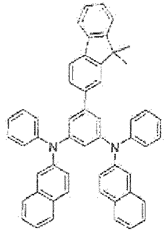

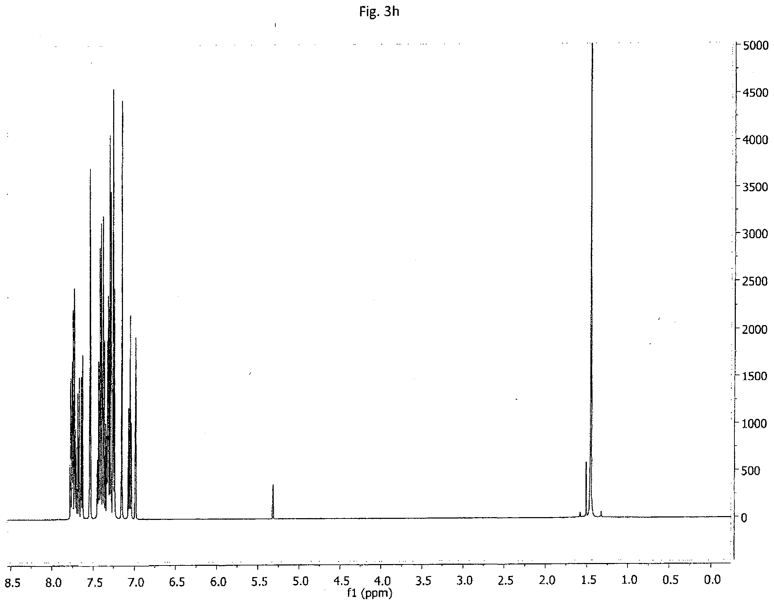

2-(3,5-dibromophenyl)-9,9-dimethyl-9H-fluorene: 3.0 g (1.0 eq, 7.01 mmol)
N-phenylnaphthalen-2-amine:3.2 g (2.1 eq, 14.71 mmol)
bis(dibenzylidenaceton)palladium: 81 mg (2 mol.%, 0.14 mmol)
tri-*tert*-butylphosphine: 43 mg (3 mol.%, 0.21 mmol)
potassium-*tert*-butoxide: 2.36 g (3.0 eq, 21.03 mmol)
toluene: 90 mL
Yield: 4.54 g (92 %)
EI-MS: m/z = 704.6 [M⁺]
¹H-NMR: see Fig. 3h

### 5-(9,9-dimethyl-9H-fluoren-2-yl)-N1,N3-di(naphthalen-2-yl)-N1,N3-di-m-tolylbenzene-1,3-diamine (FPD-9)

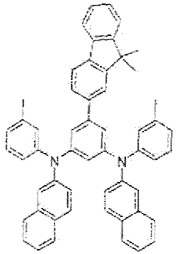

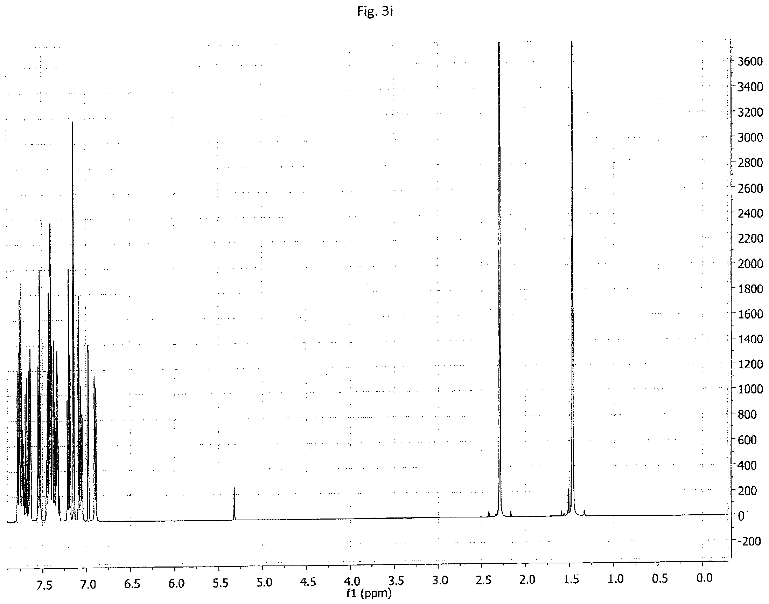

2-(3,5-dibromophenyl)-9,9-dimethyl-9H-fluorene: 3.0 g (1.0 eq, 7.01 mmol)
N-phenylnaphthalen-2-amine: 3.4 g (2.1 eq, 14.71 mmol)
bis(dibenzylidenaceton)palladium: 81 mg (2 mol.%, 0.14 mmol)
tri-*tert*-butylphosphine: 43 mg (3 mol.%, 0.21 mmol)
potassium-*tert*-butoxide: 2.36 g (3.0 eq, 21.03 mmol)
toluene: 175 mL
Yield: 5.80 g (63 %)
EI-MS: m/z = 732 [M⁺]
¹H-NMR: see Fig. 3i

### 5-(9,9-dimethyl-9H-fluoren-2-yl)-N1,N3-bis(3,5-dimethylphenyl)-N1,N3-di(naphthalen-2-yl)benzene-1,3-diamine (FPD-10)

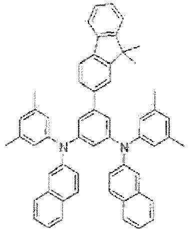

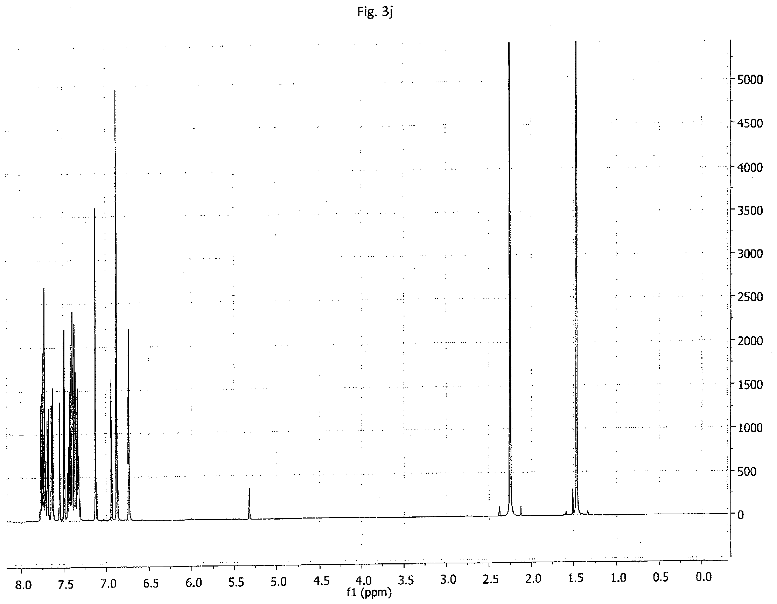

2-(3,5-dibromophenyl)-9,9-dimethyl-9H-fluorene: 3.0 g (1.0 eq, 7.01 mmol)
N-(3,5-dimethylphenyl)naphthalen-2-amine: 3.6 g (2.1 eq, 14.71 mmol)
bis(dibenzylidenaceton)palladium: 81 mg (2 mol.%, 0.14 mmol)
tri-*tert*-butylphosphine: 43 mg (3 mol.%, 0.21 mmol)
potassium-*tert*-butoxide: 2.36 g (3.0 eq, 21.03 mmol)
toluene: 150 mL
Yield: 4.96 g (65 %)
EI-MS: m/z = 760 [M⁺]
¹H-NMR: see Fig. 3j

### OLED preparation and testing

Performance testing of the new materials was carried out in bottom emitting phosphorescent organic light emitting diodes (OLED). The diodes were processed in vacuum via vapor thermal deposition of organic materials (active layers) and metals (electrodes). Shadow mask techniques were used to structure the devices (active matrix, electrodes). Four OLEDs are prepared on one substrate with an active area of 6.70 mm² each. 16 identical indium tin oxide (ITO) substrates were processed at once in a 4x4 array placed on a table which is pivotable around its vertical axe. Using shutters, each of these 16 substrates can be covered by different set of organic layers.

The ITO substrates were cleaned and put into a vapor thermal deposition unit in the 4x4 array. A reference p-doped layer (e.g. H-1 doped with D1; weight ratio (97:3) was deposited on half of these substrates for a final film thickness of 30 nm. On the other half of the plate, the studied inventive material was codeposited with the same p-dopant at the same 97:3 weight ratio and thickness. After a rotation of the plate by 90°, the second (electron blocking) layer is deposited on top of the first layer. Here, half the plate is covered with 10 nm of the reference compound (e.g., TCTA) and the other half with the same inventive material as used in the first layer (see figure 1).

The reference devices (figure 1, field D) were thus always processed together with the devices comprising the inventive materials. This approach allows assessing performance of new material in comparison with the reference independent from possible day-to-day variations of deposition rates, vacuum quality or other tool performance parameters. As each field contains 16 identically prepared OLEDs and the performance parameters were estimated for each of these 16 OLEDs, statistical evaluation of the obtained experimental results unequivocally showed the statistical significance of the observed average values reported in the Table 1.

The subsequent phosphorescent green emission layer (Merck_TMM004:Irrpy at weight ratio 9:1) was deposited with a thickness of 20 nm, followed by 20 nm Merck_TMM004 as a hole blocking layer and 25 nm E-2 layer doped with D3 (20 weight %). The cathode was prepared by vacuum deposition of 100 nm aluminum layer.

### Technical effect of the invention

Table 1 shows the experimental results obtained by the procedure described in detail in the examples below. In the experimental OLEDs tested, the hole transporting layer was doped with a p-dopant, what is symbolized with the p- symbol in the substrate/HTL/EBL column. In the table, to the compounds showing voltage lower voltage than reference, negative values were assigned in the voltage column. Oppositely, a positive value in the voltage column shows unfavourable, higher average voltage observed at the set of devices comprising inventive compound in comparison with the average voltage measured on the set of reference devices prepared under the same conditions. In the efficiency column, the average efficiency of devices comprising an inventive compound higher than the average efficiency of comparative devices is positive, whereas unfavourable lower efficiency in comparison with reference has negative sign. The last column in the table shows the arithmetic difference between the value in the efficiency column and the value in the voltage column. The resulting value was used as a benchmark for assessing the overall performance. Its positive value in at least one from the three rows shows that at least in one application - if the compound was used as an EBL, as an HTL, or in both layers - shows that in this particular case, the percentage voltage improvement has overweighed the percentage efficiency decrease or, oppositely, that the percentage efficiency improvement overweighed the undesired voltage increase, or that there was an improvement in both properties. The gained knowledge was exploited for providing new hole transporting and electron-blocking matrix materials, particularly useful in OLEDs comprising triplet emitters.

**Table 1**

| **Compound tested** | **Core substit.** | **Periphery s.** | **phosphorescent green** | | | |
|---|---|---|---|---|---|---|
| | | | substrate/HTL/EBL | voltage change [%] | Q eff change [%] | Q - voltage [%] |
| H-1 | 4,4"terphenyl | biphenyl-4-yl | ITO / p-H-1 /H-1 | -8 | -38 | -30 |
| | | biphenyl-4-yl | | | | |
| H-2 | 4,4"terphenyl | 1-naphthyl phenyl | ITO / p-H-1 / H-2 | -8 | -49 | -41 |
| | | | ITO / p-H-2 / H-2 | -8 | -50 | -42 |
| TCTA | triphenylamine | carbazole | ITO / **p-H-1 / TCTA** | 0 | | reference |
| | | | ITO / p- TCTA / TCTA | +38 | +5 | |
| FPD-1 | 9H-fluorenyl | biphenyl-4-yl phenyl | ITO / p-H-1 / MPD-1 | -11 | -5 | 6 |
| | | | ITO / **p-MPD-1** / TCTA | +3 | +5 | 2 |
| | | | ITO / **p-MPD-1/ MPD-1** | -9 | -2 | 7 |
| FPD-2 | 9H-fluorenyl | biphenyl-4-yl p-tolyl | ITO / p-H-1 / **MPD-2** | -12 | -16 | -4 |
| | | | ITO / **p-MPD-2/** TCTA | +5 | +10 | 5 |
| | | | ITO / **p-MPD-2/ MPD-2** | -11 | -8 | 3 |
| FPD-3 | 9H-fluorenyl | biphenyl-4-yl 3,5-dimethylphenyl | ITO / p-H-1 / **MPD-3** | -12 | -9 | 3 |
| | | | ITO / **p-MPD-3** / TCTA | +7 | +10 | 3 |
| | | | ITO / **p-MPD-3/ MPD-3** | -10 | -3 | 7 |
| FPD-4 | 9H-fluorenyl | biphenyl-4-yl 2,4-dimethylphenyl | ITO / p-H-1 / **MPD-4** | -11 | -1 | 10 |
| | | | ITO / **p-MPD-4** / TCTA | +6 | +5 | -1 |
| | | | ITO / **p-MPD-4/ MPD-4** | -11 | 1 | 12 |
| FPD-5 | 9H-fluorenyl | biphenyl-4-yl mesityl | ITO / p-H-1 / **MPD-5** | -11 | -2 | 9 |
| | | | ITO / **p-MPD-5** / TCTA | +8 | +12 | 4 |
| | | | ITO / **p-MPD-5/ MPD-5** | -5 | +9 | 14 |
| FPD-6 | 9H-fluorenyl | biphenyl-4-yl biphenyl-4-yl | ITO / p-H-1**/MPD-6** | -11 | -13 | -2 |
| | | | ITO / **p-MPD-6** / TCTA | 0 | +1 | 1 |
| | | | ITO / **p-MPD-6/** MPD-6 | -11 | -12 | -1 |
| FPD-7 | 9H-fluorenyl | phenyl 1-naphtyl | ITO / p-H-1 / **MPD-7** | -9 | -57 | -48 |
| | | | ITO / **p-MPD-7** / TCTA | +3 | +5 | 2 |
| | | | ITO / **p-MPD-71 MPD-7** | -4 | -54 | -50 |

Additionally, it has been found that inventive compounds are advantageous also when used as hole transporting and/or electron blocking matrices in blue fluorescent OLEDs.

The features disclosed in the foregoing description and in the claims may, both separately and in any combination, be material for realizing the invention in diverse forms thereof.

### Abbreviations used throughout the application

- OLED: organic light emitting device
- EL: electroluminescence, electroluminescent
- ETL: electron transport layer
- HTL: hole transport layer
- EBL: electron blocking layer
- HBL: hole blocking layer
- EML: emitting layer
- EIL: electron injecting layer
- HIL: hole injecting layer
- ¹H-NMR: proton magnetic resonance
- EI-MS: electron impact mass spectroscopy
- BINAP: 2,2'-bis(diphenylphosphino)-1,1'-binaphthyl
- DCM: dichloromethane
- TLC: thin layer chromatography

## Claims

1. Organic light emitting device comprising between anode and cathode at least one emitting layer comprising a phosphorescent emitter and at least one hole transporting and/or electron blocking layer comprising a compound represented by general formula (I) 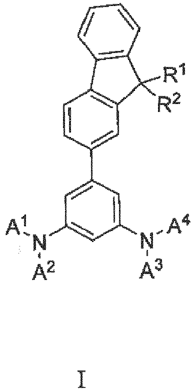
wherein R¹ and R² can be independently selected from hydrogen, C₁-C₂₀ alkyl or C₃-C₂₀ cycloalkyl, C₇-C₂₀ arylalkyl, C₆-C₁₂ aryl,
A¹, A², A³ and A⁴ are independently selected from C₆-C₂₀ aryl, and the substituents R¹ and R² may be linked so that they form a ring.

2. Device according to claim 1, wherein in each pair of aryl substituents attached to the same nitrogen, at least one aryl selected from A¹ and A² and at least one aryl selected from A³ and A⁴ are C₆-C₁₅ aryls.

3. Device according to any of claims 1-2, wherein aryl substituents A¹. A², A³ and A⁴ are C₆-C₁₅ aryls.

4. Device according to any of claims 1-3, wherein aryl substituents A¹, A², A³ and A⁴ are selected from phenyl, tolyl, xylyl, trimethylphenyl, tert-butylphenyl, 1,1'-biphenyl-yl, naphtyl and 9H-fluorenyl.

5. Device according to any of claims 1-4, wherein aryl substituents A¹, A², A³ and A⁴ are selected from 1,1 '-biphenyl-yl and 9H-fluorenyl.

6. Device according to any of claims 1-5, wherein 1,1'-biphenyl-yl is 1,1'-biphenyl-4-yl and fluorenyl is 9,9'-dimethyl-9H-fluoren-2-yl.

7. Device according to any of claims 1-6, wherein R¹ and R² are independently selected from C₁-C₁₂ alkyl.

8. Device according to any of claims 1-6, wherein at least one layer containing the compound of formula (I) is electrically doped.

9. Device according to claim 8, wherein the electrically doped layer containing compound of formula (I) is adjacent to another, electrically undoped layer comprising compound of formula (1).

10. Compound represented by general formula (I) 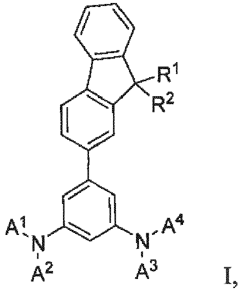
wherein R¹ and R² can be independently selected from hydrogen, C₁-C₂₀ alkyl or C₃-C₂₀ cycloalkyl, C₇-C₂₀ arylalkyl, C₆-C₁₂ aryl,
A¹, A², A³ and A⁴ are independently selected from C6-C20 aryl, and the substituents R¹ and R² may be linked so that they form a ring.

## Patentansprüche

1. Organische lichtemittierende Vorrichtung, umfassend zwischen Anode und Kathode mindestens eine emittierende Schicht, die einen phosphoreszierenden Emitter umfasst, und mindestens eine lochtransportierende und/oder elektronenblockierende Schicht, die eine durch die allgemeine Formel (I) dargestellte Verbindung umfasst, 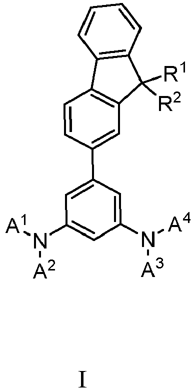
wobei R¹ und R² unabhängig ausgewählt sein können aus Wasserstoff, C₁-C₂₀-Alkyl oder C₃-C₂₀-Cycloalkyl, C₇-C₂₀-Arylalkyl, C₆-C₁₂-Aryl,
A¹, A², A³ und A⁴ unabhängig ausgewählt sind aus C₆-C₂₀-Aryl, und die Substituenten R¹ und R² so verknüpft sein können, dass sie einen Ring bilden.

2. Vorrichtung nach Anspruch 1, wobei in jedem Paar von Arylsubstituenten, die an denselben Stickstoff gebunden sind, mindestens ein Aryl, ausgewählt aus A¹ und A², und mindestens ein Aryl, ausgewählt aus A³ und A⁴, C₆-C₁₅-Aryle sind.

3. Vorrichtung nach einem der Ansprüche 1-2, wobei die Arylsubstituenten A¹, A², A³ und A⁴ C₆-C₁₅-Aryle sind.

4. Vorrichtung nach einem der Ansprüche 1-3, wobei die Arylsubstituenten A¹, A², A³ und A⁴ ausgewählt sind aus Phenyl, Tolyl, Xylyl, Trimethylphenyl, tert-Butylphenyl, 1,1'-Biphenyl-yl, Naphthyl und 9H-Fluorenyl.

5. Vorrichtung nach einem der Ansprüche 1-4, wobei die Arylsubstituenten A¹, A², A³ und A⁴ ausgewählt sind aus 1,1' -Biphenyl-yl und 9H-Fluorenyl.

6. Vorrichtung nach einem der Ansprüche 1-5, wobei 1,1'-Biphenyl-yl 1,1'-Biphenyl-4-yl ist und Fluorenyl 9,9'-Dimethyl-9H-fluoren-2-yl ist.

7. Vorrichtung nach einem der Ansprüche 1-6, wobei R¹ und R² unabhängig ausgewählt sind aus C₁-C₁₂-Alkyl.

8. Vorrichtung nach einem der Ansprüche 1-6, wobei mindestens eine Schicht, die die Verbindung der Formel (I) enthält, elektrisch dotiert ist.

9. Vorrichtung nach Anspruch 8, wobei die elektrisch dotierte Schicht, die die Verbindung der Formel (I) enthält, benachbart zu einer anderen, elektrisch undotierten Schicht ist, die die Verbindung der Formel (I) umfasst.

10. Verbindung, dargestellt durch die allgemeine Formel (I) 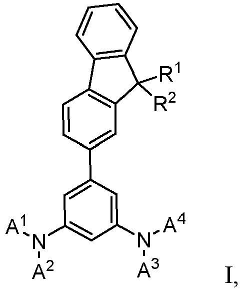
wobei R¹ und R² unabhängig ausgewählt sein können aus Wasserstoff, C₁-C₂₀-Alkyl oder C₃-C₂₀-Cycloalkyl, C₇-C₂₀-Arylalkyl, C₆-C₁₂-Aryl,
A¹, A², A³ und A⁴ unabhängig ausgewählt sind aus C₆-C₂₀-Aryl, und die Substituenten R¹ und R² so verknüpft sein können, dass sie einen Ring bilden.

## Revendications

1. Dispositif électroluminescent organique comprenant entre une anode et une cathode au moins une couche d'émission comprenant un émetteur phosphorescent et au moins une couche de transport de trous et/ou de blocage d'électrons comprenant un composé représenté par la formule générale (I) 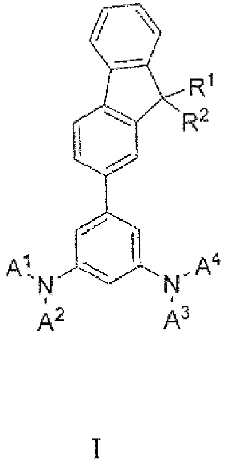
dans lequel R¹ et R² peuvent être indépendamment sélectionnés parmi un atome d'hydrogène, un groupe alkyle en C₁-C₂₀ ou cycloalkyle en C₃-C₂₀, arylalkyle en C₇-C₂₀, aryle en C₆-C₁₂,
A¹, A², A³ et A⁴ sont indépendamment sélectionnés parmi un groupe aryle en C₆-C₂₀, et les substituants R¹ et R² peuvent être liés de manière à former un cycle.

2. Dispositif selon la revendication 1, dans lequel chaque paire de substituants aryle est liée au même atome d'azote, au moins un groupe aryle sélectionné parmi A¹ et A² et au moins un groupe aryle sélectionné parmi A³ et A⁴ sont des groupes aryle en C₆-C₁₅.

3. Dispositif selon l'une quelconque des revendications 1 à 2, dans lequel les substituants aryle A¹, A², A³ et A⁴ sont des groupes aryle en C₆-C₁₅.

4. Dispositif selon l'une quelconque des revendications 1 à 3, dans lequel les substituants aryle A¹, A², A³ et A⁴ sont sélectionnés parmi des groupes phényle, tolyle, xylyle, triméthylphényle, tert-butylphényle, 1,1'-biphényl-yl, naphtyle et 9H-fluorényle.

5. Dispositif selon l'une quelconque des revendications 1 à 4, dans lequel les substituants aryle A¹, A², A³ et A⁴ sont sélectionnés parmi le 1,1'-biphényl-yl et le 9H-fluorényle.

6. Dispositif selon l'une quelconque des revendications 1 à 5, dans lequel le 1,1'-biphényl-yl est le 1,1'-biphényl-4-yl et le groupe fluorényle est le 9,9'-diméthyl-9H-fluoren-2-yl.

7. Dispositif selon l'une quelconque des revendications 1 à 6, dans lequel R¹ et R² sont indépendamment sélectionnés parmi un groupe alkyle en C₁-C₁₂.

8. Dispositif selon l'une quelconque des revendications 1 à 6, dans lequel au moins une couche contenant le composé de formule (I) est électriquement dopée.

9. Dispositif selon la revendication 8, dans lequel la couche électriquement dopée contenant un composé de formule (I) est adjacente à une autre couche non électriquement dopée comprenant un composé de formule (I).

10. Composé représenté par la formule générale (I) 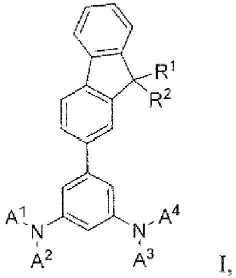
dans lequel R¹ et R² peuvent être indépendamment sélectionnés parmi un atome d'hydrogène, un groupe alkyle en C₁-C₂₀ ou cycloalkyle en C₃-C₂₀, arylalkyle en C₇-C₂₀, aryle en C₆-C₁₂,
A¹, A², A³ et A⁴ sont indépendamment sélectionnés parmi un groupe aryle en C₆-C₂₀, et les substituants R¹ et R² peuvent être liés de manière à former un cycle.
